(19) Europäisches Patentamt
European Patent Office
Office européen des brevets

(11) EP 2 832 719 B1

(12) EUROPEAN PATENT SPECIFICATION

(45) Date of publication and mention of the grant of the patent:
**01.02.2017 Bulletin 2017/05**

(51) Int Cl.:
***C07C 59/64*** *(2006.01)* ***A61K 8/49*** *(2006.01)*
***A61Q 19/02*** *(2006.01)*

(21) Application number: **14179291.1**

(22) Date of filing: **31.07.2014**

# (54) Melanogenesis-inhibitory constituents of Ligusticum sinense

Bestandteile des Ligusticum sinense als Melanogenese Inhibitoren

Constituants de Ligusticum sinense possédant une activité inhibitoire de la mélanogenèse

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2013 TW 102127636**

(43) Date of publication of application:
**04.02.2015 Bulletin 2015/06**

(73) Proprietor: **Lee, Ching Kuo**
**Xindian City, Taipei 231 (CN)**

(72) Inventors:
- **Lee, Ching-Kuo**
  **Taipei County 231 (TW)**
- **Chu, Yi-Tzu**
  **Taipei City 104 (TW)**
- **Lai, Horng-Ji**
  **Taipei City 106 (TW)**
- **Liang, Wen-Li**
  **New Taipei City 231 (TW)**

(74) Representative: **Bailey, Sam Rogerson et al**
**Mewburn Ellis LLP**
**City Tower**
**40 Basinghall Street**
**London EC2V 5DE (GB)**

(56) References cited:
**EP-A1- 1 330 999** **JP-A- 2000 247 874**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 832 719 B1

## Description

### BACKGROUND

Technical Field

The disclosure relates to melanogenesis-inhibitory constituents of *Ligusticum sinense.*

Description of Related Art

Melanin is primarily responsible for skin color, but also plays an important role in preventing sunburn. A physiological response of human skin will produce melanin to prevent sunburn when exposed to UV light. The melanogenesis is regulated by enzyme, such as tyrosinase and related proteins. Therefore, inhibition of the tyrosinase catalyzed steps of melanogenesis to reduce melanogenesis is the most common skin whitening method. Since most women want to avoid UV tanning in Asia, screening a compound that can inhibit tyrosinase or melanogenesis from natural resources would be useful. JP 2000 247874 A discloses a melanogenesis inhibitor for external use for skin suitable for bleaching.

### SUMMARY

In one aspect, the present invention is directed to an isolated compound III having a chemical structure as follows.

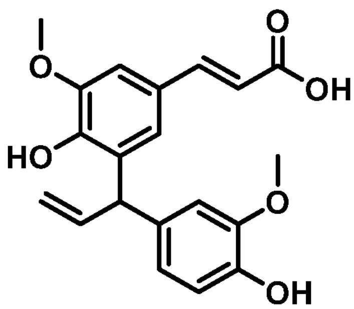

(III)

In another aspect, the present invention is directed to a composition for use in the therapeutic inhibition of melanogenesis, the composition comprising an effective amount of a compound having at least one of the chemical structures shown below for the application on skin to inhibit melanogenesis.

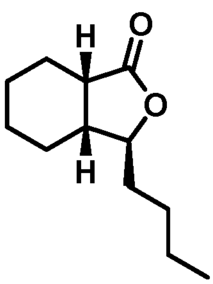

(I)

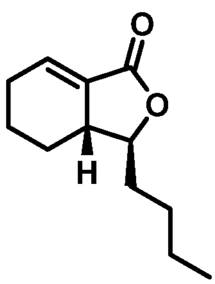

(II)

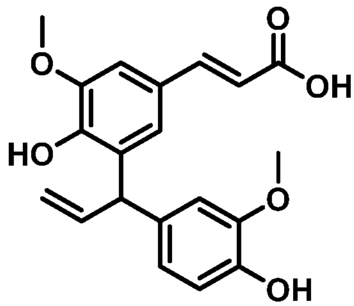

(III)

### BRIEF DESCRIPTION OF THE DRAWINGS

Fig. 1 is an ESI-MS spectrum of the compound III.
Fig. 2 is an IR spectrum of the compound III.

Fig. 3 is a $^1$H NMR (500 MHz, acetone-$d_6$) spectrum of the compound III.
Fig. 4 is a $^{13}$C NMR (125 MHz, acetone-$d_6$) spectrum of the compound III.
Fig. 5 is a HSQC (acetone-$d_6$) spectrum of the compound III.
Fig. 6 is a HMBC (acetone-$d_6$) spectrum of the compound III.
Fig. 7 is a NOESY (acetone-$d_6$) spectrum of the compound III.
Fig. 8A is an experimental result of the cell viability of B16-F10 murine melanoma cells.
Fig. 8B is an experimental result of the melanin content in B16-F10 murine melanoma cells.
Fig. 8C is an experimental result of the cell viability of Human Epidermal Skin Equivalents (Leiden epidermal models; LEMs).

## DETAILED DESCRIPTION

*Ligusticum sinense* Oliver is a traditional Chinese medicine, and is also called Chinese Lovage. Two thousand years ago, Chinese people used it to expel wind-cold, relieve pain, and whiten and moisturize skin. In the following experiment, a methanol extract of *Ligusticum sinense* Oliver is purified and identified. Then, the purified compounds from the methanol extract underwent assays of activity of inhibiting tyrosinase, cell viability of melanocytes, and melamine content in melanocytes to find compounds that can whiten skin.

### Preparation and Screening of *Ligusticum sinense* Oliver's Extract

9.9 kg rhizome of *Ligusticum sinense* Oliver was smashed, and 40 L of methanol was used to cold dip the powder of *Ligusticum sinense* Oliver to obtain an extract solution. The extracting process above was repeated 3 times. After concentrated under a reduced pressure, 1758 g of a crude extract was obtained, and yield was 17.8%.

1458 g of the crude extract was partitioned by 1: 1 (v/v) of water and ethyl acetate (EA) for 3 times. The EA extracted layer was collected. The weight of the obtained EA extracted layer was 415 g.

250 g of EA extracted layer was mixed with 375 g of 0.2 mm silica gel and absorbed by the silica gel. 3350 g of 40-63 μm silica gel was used to fill a column for preparing chromatography. Next, various volume ratios of n-hexane (Hex) and EA (Hex/EA=95/5, 90/10, 80/20, 60/40, 40/60, v/v) was used as eluent. Each volume ratio of eluent used was 20 L, and each 500 mL of the eluent was collected in one bottle. Each bottle was preliminarily analyzed by thin layer chromatography (TLC). The detailed related information above was listed in the Table 1 below.

Table 1: Solvent used to collect eleuent of column chromatography

| Parts | Collect Ranges | Solvent Volume Ratio of Eluent |
|---|---|---|
| 1 | Bottles 1-39 (total 39 bottles) | 95/5 (v/v) Hex/EA |
| 2 | Bottles 40-77 (total 38 bottles) | 90/10 (v/v) Hex/EA |
| 3 | Bottles 78-115 (total 38 bottles) | 80/20(v/v) Hex/EA |
| 4 | Bottles 116-155 (total 40 bottles) | 60/40 (v/v) Hex/EA |
| 5 | Bottles 156-196 (total 41 bottles) | 40/60 (v/v) Hex/EA |

According to the analysis result of TLC, the total eluent was divided into 5 parts and then dried respectively. Next, high performance liquid chromatography (HPLC) was used to perform purification steps.

After performing the HPLC purification steps, 24 compounds were isolated, and three of them (compound I, II, III) have inhibitory activity of melanogenesis. The chemical structures and the HPLC purification conditions are listed Table 2 below. The compound I (Bartschat, D., Beck, T., & Mosandl, A. (1997) Stereoisomeric flavor compounds. 79. Simultaneous enantioselective analysis of 3-butylphthalide and 3-butylhexahydrophthalide stereoisomers in celery, celeriac, and fennel. Journal of Agricultural and Food Chemistry, 45, 4551-4557) and the compound II (Oguro, D., & Watanabe, H. (2011) Synthesis and sensory evaluation of all stereoisomers of sedanolide. Tetrahedron, 67, 777-781) are known compounds, and the compound III is a new compound.

Table 2: The chemical structures and HPLC separation conditions of the compounds I, II, and III.

| compound | Chemical structure | Separation conditions of HPLC* |
|---|---|---|
| I | 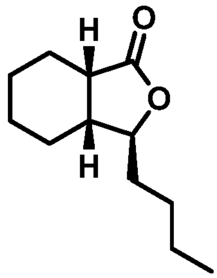 | Mobile phase: n-Hexane/EA = 96/4<br>Retention time: 23.5 min |
| II | 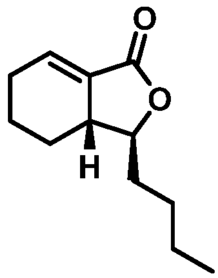 | Mobile phase: n-Hexane/EA = 95/5<br>Retention time: 19.3 min |
| III | 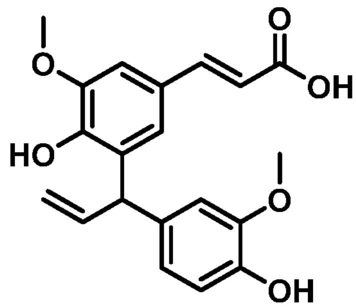 | Mobile phase: n-Hexane/EA = 53/47<br>Retention time: 13.2 min |
| * Column used for HPLC was Hibar® Fertigäute semi-preparation column (10 mm × 250 mm) using RI detector. The flow rates of the mobile phase were all 3.0 mL/min. | | |

The physical properties and spectrum data of the compound I (3-Butylhexahydrophthalide) was listed below. Colorless oil. Formula: $C_{12}H_{20}O_2$. **ESI-MS** $[M+H]^+$ *m/z*: 197.2. $[\alpha]_D^{25}$ − 13.4° (*c* 0.13, $CHCl_3$). **IR (neat)** $\upsilon$ max $cm^{-1}$: 2929, 2861, 1766, 1453, 1369, 1128. **$^1$H□NMR** (500 MHz, Chloroform-*d*) $\delta_H$: 0.89 (3H, t, *J* = 7.0 Hz), 1.20 - 1.76 (13H, m), 1.94 (1 H, m), 2.16 (1 H, m), 2.67 (1 H, m), 4.08 (1 H, m). **$^{13}$C-NMR** (125 MHz, Chloroform-*d*) $\delta_C$: 14.1, 22.7, 23.1, 23.3, 23.4, 27.5, 28.2, 33.1, 38.7, 39.6, 84.0, 178.7.

The physical properties and spectrum data of the compound **II** (Neocnidilide) was listed below. Colorless oil. Formula: $C_{12}H_{18}O_2$. **ESI-MS** $[M+H]^+$ *m/z*: 195.2. $[\alpha]_D^{25}$ − 60.1° (*c* 0.37, $CHCl_3$). **IR (neat)** $\upsilon_{max}$ $cm^{-1}$: 2934, 2865, 1744, 1453, 1329, 1220, 1026. **$^1$H□NMR** (500 MHz, Chloroform-*d*) $\delta_H$: 0.89 (3H, t, *J* = 7.0 Hz), 1.10 - 2.05 (10H, m), 2.17 (1 H, m), 2.31 (1 H, m), 2.46 (1 H, m), 3.93 (1 H, ddd, *J* = 8.9, 7.6, 5.5 Hz), 6.74 (1 H, dd, *J* = 6.7, 3.1 Hz). **$^{13}$C-NMR** (125 MHz, Chloroform-d) $\delta_C$: 14.1, 20.9, 22.7, 25.2, 25.5, 27.7, 34.5, 43.2, 85.6, 131.3, 135.5, 170.5.

The physical properties and spectrum data of the compound **III** (5-[3-(4-Hydroxy-3-methoxyphenyl)allyl]ferulic acid) was listed below. Colorless viscid oil. Formula: $C_{20}H_{20}O_6$. **ESI-MS** $[M-H]^-$ m/z: 355.3. $[\alpha]_D^{25}$ + 5.6° (*c* 0.12, $CH_3OH$). **UV (MeOH)** $\lambda_{max}$ nm (log $\varepsilon$): 288 (3.9), 314 (3.8). **IR (neat)** $\upsilon_{max}$ $cm^{-1}$: 3444, 2935, 1633, 1509, 1434, 1376, 1269, 1153. **$^1$H□NMR** (500 MHz, Acetone-$d_6$) $\delta_H$: 3.77 (3H, s, 3'-$OCH_3$), 3.92 (3H, s, 3-$OCH_3$), 4.99 (1 H, ddd, *J* = 17.1, 1.8, 1.8 Hz, H-9'$_{trans}$), 5.10 (1 H, br d, *J* = 7.6 Hz, H-7'), 5.15 (1 H, ddd, *J* = 10.1, 1.8, 1.8 Hz, H-9'$_{cis}$), 6.33 (1 H, d, *J* = 15.9 Hz, H-8), 6.40 (1 H, ddd, *J* = 17.1, 10.1, 7.6 Hz, H-8'), 6.69 (1H, dd, J= 7.9, 1.8 Hz, H-6'), 6.74 (1H, d, *J* = 7.9 Hz, H-5'), 6.87 (1 H, d, *J* = 1.8 Hz, H-2'), 7.07 (1 H, d, *J* = 1.8 Hz, H-6), 7.22 (1 H, d, *J* = 1.8 Hz, H-2), 7.56 (1 H, d, *J* = 15.9

Hz, H-7). **$^{13}$C-NMR** (125 MHz, Acetone-*d*$_6$) $\delta_C$:48.2 (C-7'), 56.4 (3'-$OCH_3$), 56.6 (3-$OCH_3$), 108.9 (C-2), 113.2 (C-2'), 115.6 (C-5'), 115.9 (C-9'), 116.1 (C-8), 121.8 (C-6'), 123.8 (C-6), 126.7 (C-1), 131.2 (C-5), 135.3 (C-1'), 141.5 (C-8'), 146.1 (C-4'), 146.2 (C-7), 147.2 (C-4), 148.2 (C-3'), 148.6 (C-3), 168.4 (C-9).

**Chemical Structure Analysis of Compound III**

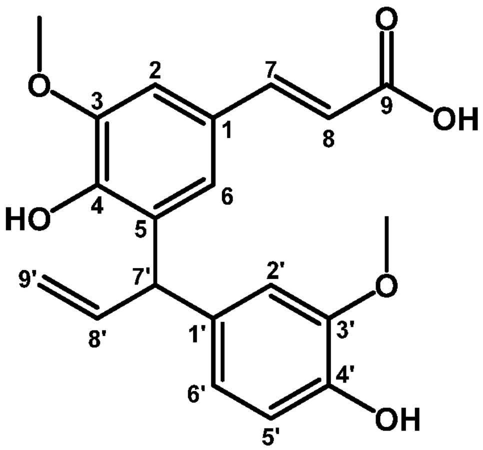

(III)

The obtained compound III is colorless viscid oil. Fig. 1 is an ESI-MS spectrum of the compound III. From Fig. 1, it is observed that the measured m/z value of $[M-H]^-$ was 355.3. Further from HR-ESI-MS, the measured m/z value of $[M-H]^-$ was 355.1080, and the calculated m/z value of $C_{20}H_{20}O_6$ was 355.1182. Therefore, the chemical formula of the compound III was $C_{20}H_{20}O_6$, and the unsaturation degree of the compound III was 11.

Fig. 2 is an IR spectrum of the compound III. The IR spectrum of the neat compound III has an O-H peak at 3444 $cm^{-1}$ (peak 12), a C=C peak at 1633 $cm^{-1}$ (peak 9), and a benzene C=C peak at 1509 $cm^{-1}$ (peak 8).

Fig. 3 is a $^1$H NMR (500 MHz, acetone-*d*$_6$) spectrum of the compound III. In Fig. 3, a set of signals located at $\delta_H$ 7.07 (1 H, d, *J* = 1.8 Hz) and 7.22 (1 H, d, *J* = 1.8 Hz) were belong to meta-coupling protons of a benzene ring. Therefore, a 4-substitued benzene ring might be presented in the compound III. A set of signals located at $\delta_H$ 6.69 (1 H, dd, *J* = 7.9, 1.8 Hz), $\delta_H$ 6.74 (1 H, d, *J* = 7.9 Hz), and 6.87 (1 H, d, *J* = 1.8 Hz) were belong to ABX coupling protons of a benzene ring. Therefore, a 3-substituted benzene ring might be presented in the compound III. A set of signals located at $\delta_H$ 6.33 (1 H, d, *J* = 15.9 Hz) and 7.56 (1 H, d, *J* = 15.9 Hz) were belong to *trans* protons of a double bond. A set of signals located at $\delta_H$ 4.99 (1 H, ddd, *J* = 17.1, 1.8, 1.8 Hz), 5.15 (1 H, ddd, *J* = 10.1, 1.8, 1.8 Hz) and 6.40 (1H, ddd, *J* = 17.1, 10.1, 7.6 Hz) were belong to a terminal protons of a double bond. Moreover, the signal at $\delta_H$ 6.40 coupled to the signal at $\delta_H$ 5.10 (1 H, br d, *J* = 7.6 Hz), which was inferred to belong to a methine group. Therefore, a methine group connecting to a terminal double bond (i.e. $-CH-CH=CH_2$) might presented in the compound III. Hoverer, it was not sure that whether the signal at $\delta_H$ 5.10 was belonged to an oxymethine group, a double bond, or other types of protons. A set of signals located at $\delta_H$ 3.77 (3H, s) and $\delta_H$ 3.92 (3H, s) were belonged to 2 methoxy ($-OCH_3$) group.

Fig. 4 is a $^{13}$C NMR (125 MHz, acetone-*d*$_6$) spectrum of the compound III. From $^{13}$C NMR and DEPT spectrum, it is observed that there were 8 quaternary carbons, 9 tertiary carbons (methine group), 1 secondary carbon (methylene group), and 2 primary carbons (methyl group). There were 20 carbons in total. A signal at $\delta_C$ 168.4 (s) was belonged to a carbonyl group of a carboxylic acid group. Sixteen signals at $\delta_C$ 108.9 (d), 113.2 (d), 115.6 (d), 115.9 (t), 116.1 (d), 121.8 (d), 123.8 (d), 126.7 (s), 131.2 (s), 135.3 (s), 141.5 (d), 146.1 (s), 146.2 (d), 147.2 (s), 148.2 (s), 148.6 (s) were belonged to two double bonds and two benzene rings, and one of the double bonds was a terminal double bonds. Signals at $\delta_C$ 56.4 (q) and 56.6 (q) were belonged to 2 methoxy groups. A signal at $\delta_C$ 48.2 (d) was belonged to a methine group, but not an oxymethine group or a double bond.

Since the chemical shifts difference ($\delta_H$ 6.33 and 7.56) of the *trans* double bond was large, together with the carbonyl group ($\delta_C$ 168.4) of the carboxylic acid group, and the multi-substituted benzene rings, methoxy groups, and disappeared hydroxyl groups, it was inferred that the compound III has a structure of a ferulic acid. That is, the compound III was a derivative of ferulic acid.

Next, HSQC and HMBC experiments were performed to determine the positions of each functional group. Fig. 5 is a HSQC (acetone-*d*$_6$) spectrum of the compound III. In Fig. 5, it can be seen that signals at $\delta_H$ 5.10 and $\delta_C$ 48.2

have cross peaks. Therefore, it can be sure that $\delta_H$ 5.10 was connected to $\delta_C$ 48.2.

Fig. 6 is a HMBC (acetone-$d_6$) spectrum of the compound III. From Fig. 6, it can be observed that $\delta_H$ 5.10 was related to $\delta_C$ 113.2, 115.9, 121.8, 123.8, 131.2, 135.3, 141.5, and 147.2. Therefore, it can be sure that the methine group of $\delta_H$ 5.10 connected to 2 benzene rings and a terminal double bond. The position of the methine group of $\delta_H$ 5.10 was at C-7', and the methine group of $\delta_H$ 5.10 connected to the carbons at positions C-5 and C-1' of the two benzene rings, and the carbon at the position C-8' of the terminal double bond. $\delta_H$ 7.56 was related to $\delta_C$ 108.9, 116.1, 123.8, 126.7, and 168.4. Therefore, it can be sure that a terminal of the *trans* double bond at positions C-7 and C-8 was connected to a carbon C-9 of a carboxylic acid group. The other terminal of the *trans* double bond at positions C-7 and C-8 was connected to a carbon C-1 of a benzene ring. $\delta_H$ 3.77 was related to $\delta_C$ 148.2, and $\delta_H$ 3.92 was related to $\delta_C$ 148.6. Therefore, it can be sure that the methoxy group of $\delta_H$ 3.77 was connected to C-3', and the methoxy group of $\delta_H$ 3.92 was connected to C-3. The two methoxy groups above were respectively located at C-4 and C-4'.

Fig. 7 is a NOESY (acetone-$d_6$) spectrum of the compound III. NOESY experiment was used to further confirm the positions of the methoxy groups. The result shows that $\delta_H$ 3.77 (3H, s) was related to $\delta_H$ 6.87 (1 H, d, $J$ = 1.8 Hz). Therefore, it can be sure that the methoxy group of $\delta_H$ 3.77 was connected to C-3', and the methoxy group of $\delta_H$ 3.92 was connected to C-3. The data about $^{13}C$ NMR, DEPT, $^{1}H$ NMR, and HMBC were listed in the Table 3 below.

Table 3: NMR data of the compound III

| positions | $^{13}C$ NMR* | $^{1}H$ NMR | HMBC |
|---|---|---|---|
| | $\delta_C$ (multi.) | $\delta_H$ (multi., $J$ in Hz) | H→C |
| 1 | 126.7 (s) | | |
| 2 | 108.9 (d) | 7.22 (d, 1.8) | C-1, C-3, C-4, C-6 |
| 3 | 148.6 (s) | | |
| 4 | 147.2 (s) | | |
| 5 | 131.2 (s) | | |
| 6 | 123.8 (d) | 7.07 (d, 1.8) | C-2, C-4, C-7' |
| 7 | 146.2 (d) | 7.56 (d, 15.9) | C-1, C-2, C-6, C-8, C-9 |
| 8 | 116.1 (d) | 6.33 (d, 15.9) | C-1, C-7, C-9 |
| 9 | 168.4 (s) | | |
| 1' | 135.3 (s) | | |
| 2' | 113.2 (d) | 6.87 (d, 1.8) | C-1', C-3', C-4', C-6', C-7' |
| 3' | 148.2 (s) | | |
| 4' | 146.1 (s) | | |
| 5' | 115.6 (d) | 6.74 (d, 7.9) | C-1', C-3', C-4' |
| 6' | 121.8 (d) | 6.69 (dd, 7.9, 1.8) | C-2', C-4', C-7' |
| 7' | 48.2 (d) | 5.10 (br d, 7.6) | C-4, C-5, C-6, C-1', C-2', C-6', C-8', C-9' |
| 8' | 141.5 (d) | 6.40 (ddd, 17.1, 10.1, 7.6) | C-5, C-1', C-7' |
| 9' | 115.9 (t) | 4.99 (ddd, 17.1, 1.8, 1.8) | C-7', C-8' |
| | | 5.15 (ddd, 10.1, 1.8, 1.8) | C-7' |
| 3-$OCH_3$ | 56.6 (q) | 3.92 (3H, s) | C-3 |
| 3'-$OCH_3$ | 56.4 (q) | 3.77 (3H, s) | C-3' |
| * The multiplicity of $^{13}C$ NMR signals was determined by DEPT experiment. | | | |

From the data above, it can be sure that the compound III was 5-[3-(4-Hydroxy-3-methoxyphenyl)allyl]ferulic acid.

**Assay of Tyrosinase Activity**

The source of the tested tyrosinase was from mushroom, and the substrate of the tyrosinase was L-tyrosine. The experiment was performed by the method below. First, 40 μL of pH 6.8 phosphate buffer solution, 40 μL of various sample solution (samples in 0.07M pH6.8 phosphate buffer solution containing 0.1 vol% of DMSO), and 40 μL of tyrosinase (250 unit/mL, dissolved in pH 6.8 phosphate buffer solution at a concentration of 0.07 M) were sequentially and respectively added into 96-wells plate. The sample solutions above respectively contain the compounds I, II, III, and kojic acid. The group added with the sample solution containing kojic acid was used as positive control (PC). A group without adding the sample solution was used as control (C).

The mixtures described above were thermostat at 37°C for 10 minutes. Then, 80 μL of tyrosine solutions (0.4 mg/mL, dissolved in 0.07M pH6.8 phosphate buffer solution) were respectively added into each mixture and thermostat for another 20 minutes to perform reaction. The amount of the obtained product L-DOPA of each mixture was measured at 475 nm to get absorbance of each sample. Therefore, the tyrosinase activity was calculated by the formula below.

$$\text{Tyrosinase activity (\%)} = [(A - B)/(C - D)] \times 100$$

The symbol A above is the absorbance of groups containing a sample and tyrosinase. The symbol B above is the absorbance of groups containing a sample but not tyrosinase. The symbol C above is the absorbance of groups containing tyrosinase but not a sample. The symbol D above is the absorbance of groups does not contain both a sample and tyrosinase.

The results of the assay showed that the compounds I, II, and III do not show obvious inhibitory effect to the tyrosinase activity.

**Viability Test of B16-F10 Murine Melanoma Cells**

In this test, B16-F10 murine melanoma cells (BCRC No. 60031) were used.

First, the B16-F10 murine melanoma cells were cultured in Dulbecco's Modified Eagle's Medium (abbreviated as DMEM, which contains 90% (v/v) DMEM, 10% (v/v) fetal bovine serum), and additionally added 1%(v/v) Penicillin-Streptomycin Solution. Then, the cells in the mediums were incubated under 5% $CO_2$/air at 37 °C in 70% of humidity. The medium was exchanged every two days. When the cells grew to a density of 80 - 90%, the cells were treated by trypsin to desorb the cells from the walls of containers. Next, haemocytometer (Neubauer Improved., Marienfeld, Germany) was used to count the number of the cells. Proper amount of cells were then transplanted into cell culture plates to prepare for cell viability tests.

Cell viability rate was determined by MTT assay. MTT assay was performed by using lactate dehydrogenase in the mitochondria of living cells to reduce yellow 3-(4,5-dimethylthiazol-2-yl)-2,5-diphenyl tetrazolium bromide (MTT) to purple formazan product. Therefore, the amount of the purple formazan product is proportional to the number of the living cells and thus can be determined by spectrophotomter.

In a 12-wells cell plate, $1 \times 10^3$ cells were implanted into each well and stayed for 24 hours to let the cells attach to the wall of the well. Then, the compounds I, II, III and arbutin (positive control; PC) were respectively added into each wells and cultured for 72 hours. Next, MTT dye (Applichem Co., Denmark) dissolved in 0.5 mg/ml PBS solution was added to each well to react for 2 hours. The obtained purple formazan product was dissolved in 400 μL of DMSO, and then the absorbance of the purple DMSO solution was analyzed by microplate reader to get the absorbance at 570 nm. The cell viability rate was determined by the formula below.

$$\text{Cell viability rate (\%)} = (\text{Absorbance of test group/ absorbance of blank group}) \times 100\%$$

Fig. 8A is an experimental result of the cell viability of B16-F10 murine melanoma cells. From Fig. 8A, it is observed that compounds I and II did not affect the cell viability, and the compound III had a slight tendency to inhibit cell growth at a concentration of 100 μM. Basically, compounds I, II and III did not have cytotoxicity to the B16-F10 murine melanoma cells.

**Analysis of Melanin Content in B16-F10 Murine Melanoma Cells**

The melanin content in B16-F10 murine melanoma cells was analyzed by a modified Hosoi method disclosed in 1985 (Hosoi J, Abe E, Suda T, Kuroki T, Regulation of melanin synthesis of B16 mouse melanoma cells by 1 alpha, 25-dihydroxyvitamin D3 and retinoic acid. Cancer Res. 1985; 45(4):1474-8).

First, in a 12-wells cell plate, $1 \times 10^3$ cells were implanted into each well and stayed for 24 hours to let the cells attach to the wall of the well. Various concentrations (25, 50, and 100 $\mu$M) of the compounds I, II, III, and arbutin (positive control; PC) were respectively added into each wells, and 100 nM of $\alpha$-melanocyte stimulating hormone ($\alpha$-MSH) was subsequently adaded. The cells were cultured for 72 hours. After the treatment above, cells were washed by PBS, and then treated by trypsin to desorb the cells. 150 $\mu$L of 1 N NaOH solution containing 10% DMSO was then added and heated in a 80°C water bath for 1 hour. After cooling to room temperature, the samples were centrifuge at 5000 rpm for 10 minutes. The upper solution was transferred into a 96-wells plate and analyzed by a microplate reader at 405 nm. The absorbance at 405 nm for each sample was read to calculate the melanin content of each sample.

Fig. 8B is an experimental result of the melanin content in B16-F10 murine melanoma cells. From Fig. 8B, it is observed that compounds I, II and III can decrease the melanin content in the melanoma cells without affecting the cell viability rate. The $IC_{50}$ of the compounds I, II and III for inhibiting the melanogenesis were 100.1 $\pm$ 12.9 $\mu$M, 31.1 $\pm$ 6.8 $\mu$M, and 78.9 $\pm$ 8.1 $\mu$M, respectively. Therefore, it is observed that the compound II has the best inhibitory activity for melanogenesis.

**Viability Test of Human Epidermal Skin Equivalents**

In this test, fresh mamma reduction surplus skin of a single female individual (D002, 59 years, Fitzpatrick skin type I/II) was used for isolation of normal human epidermal keratinocytes (NHEKs).

Leiden epidermal models (LEMs) were generated by seeding NHEKs on an inert filter insert. The NHEKs in LEMs were incubated overnight under submerged conditions in keratinocyte medium. Within four days, fetal bovine serum was gradually omitted and the NHEKs in LEMs were cultured serum-free at the air-liquid interface for 7 days, while culture medium was refreshed twice a week.

Viability assays were performed by adding 0,5 mL of 1 mg/mL MTT to each of the NHEKs in LEMs for 3h, after 24 hours exposure to the test articles (compounds II, III, and DMSO (negative control)). The precipitated blue formazan product was extracted from the cells within 2 hours with 0.5 mL isopropanol per well. The concentration of formazan was measured by determining the OD at 570 nm using a Tecan Infinite F50 microplate reader.

Fig. 8C is an experimental result of the cell viability of the NHEKs in Leiden epidermal models. From Fig. 8C, it is observed that compounds II and III did not affect the cell viability at a concentration of 10-100 $\mu$M. The cell viability of compounds II is 97% (10 $\mu$M), and 92% (100 $\mu$M). The cell viability of compounds III is 98% (10 $\mu$M), 104 (50 $\mu$M), and 106 % (100 $\mu$M). Accordingly, compounds II and III did not have cytotoxicity to the NHEKs in the Leiden epidermal models.

## Claims

1. An isolated compound III having a chemical structure as follows:

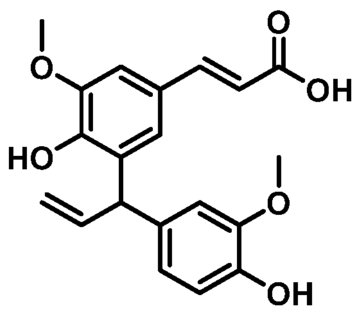

(III)

2. A composition for use in the therapeutic inhibition of melanogenesis, the composition comprising:

an effective amount of a compound having at least one of the chemical structures shown below for the application

on skin to inhibit melanogenesis.

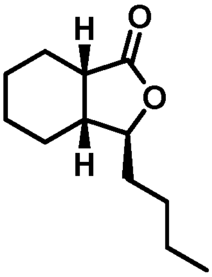

(I)

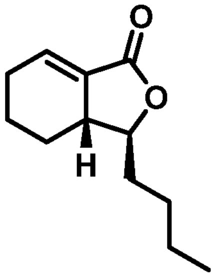

(II)

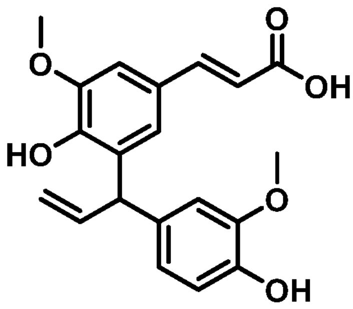

(III)

**Patentansprüche**

1. Isolierte Verbindung III mit einer chemischen Struktur wie folgt:

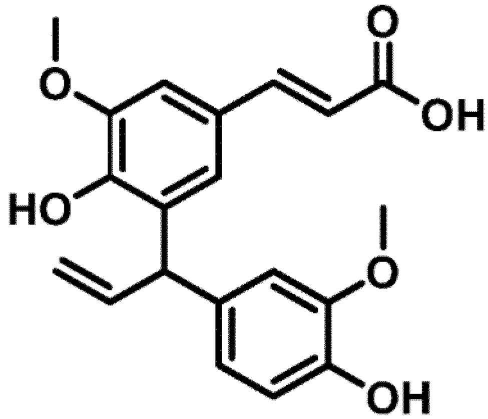

(III)

2. Zusammensetzung zur Verwendung bei der therapeutischen Hemmung von Melanogenese, wobei die Zusammensetzung Folgendes umfasst:

   eine wirksame Menge einer Verbindung mit zumindest einer der nachstehend dargestellten chemischen Strukturen zur Anwendung auf der Haut, um Melanogenese zu hemmen.

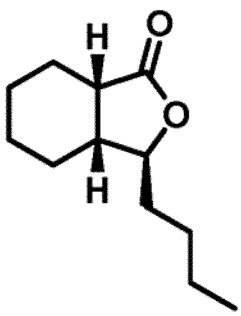

(I)

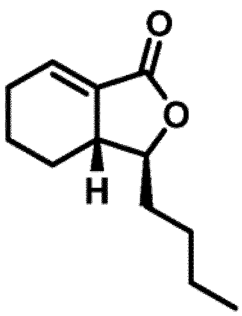

(II)

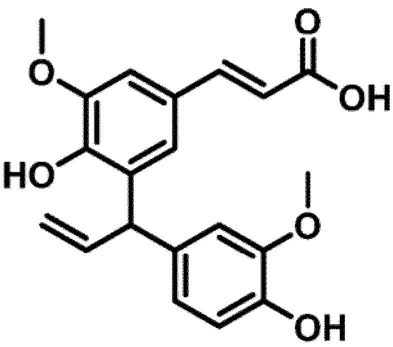

(III)

## Revendications

1. Composé III isolé ayant la structure chimique suivante :

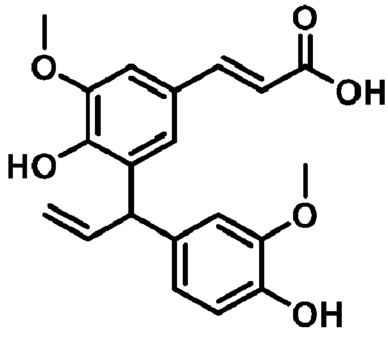

(III)

2. Composition pour utilisation dans l'inhibition thérapeutique de la mélanogenèse, la composition comprenant une quantité efficace d'un composé ayant au moins l'une des structures chimiques indiquées ci-dessous pour l'application sur la peau afin d'inhiber la mélanogenèse.

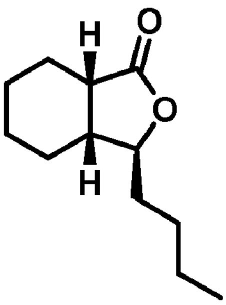

(I)

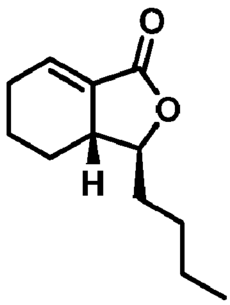

(II)

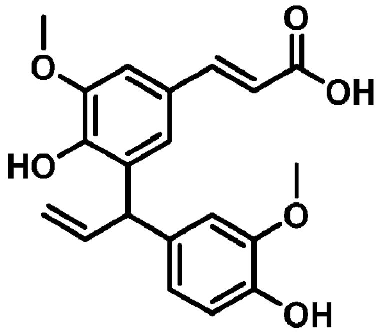

(III)

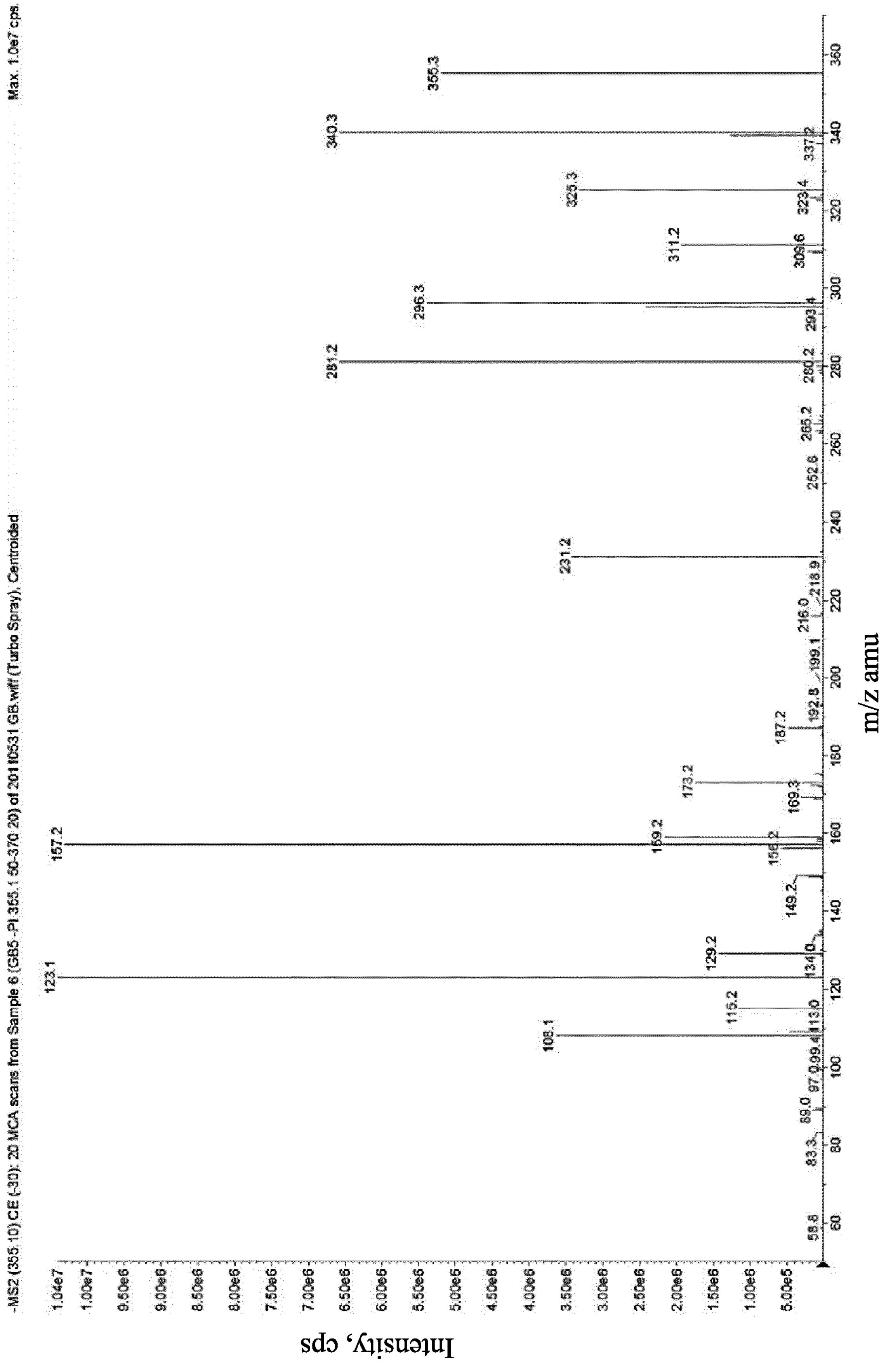
-MS2 (355.10) CE (-30): 20 MCA scans from Sample 6 (GB5 -PI 355.1 50-370 20) of 20110531 GB.wiff (Turbo Spray), Centroided
Max. 1.0e7 cps.
Intensity, cps
m/z amu
355.3
340.3
337.2
325.3
323.4
311.2
309.6
296.3
293.4
281.2
280.2
265.2
252.8
231.2
218.9
216.0
199.1
192.8
187.2
173.2
169.3
159.2
157.2
156.2
149.2
134.0
129.2
123.1
115.2
113.0
108.1
99.4
97.0
89.0
83.3
58.8

Fig. 1

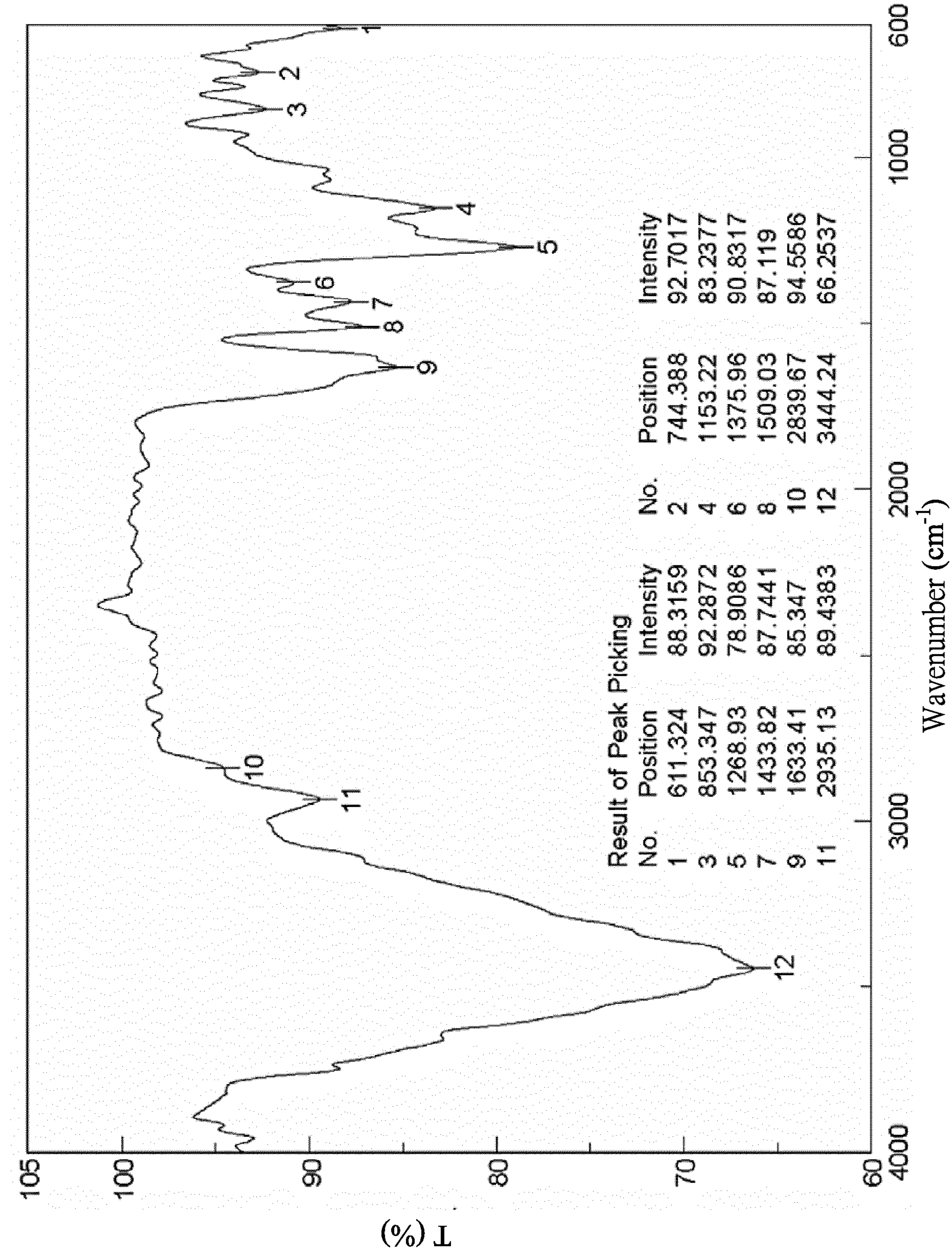
T (%)
105
100
90
80
70
60
4000
3000
2000
1000
600
Wavenumber (cm-1)
1
2
3
4
5
6
7
8
9
10
11
12
Result of Peak Picking
No. Position Intensity No. Position Intensity
1 611.324 88.3159 2 744.388 92.7017
3 853.347 92.2872 4 1153.22 83.2377
5 1268.93 78.9086 6 1375.96 90.8317
7 1433.82 87.7441 8 1509.03 87.119
9 1633.41 85.347 10 2839.67 94.5586
11 2935.13 89.4383 12 3444.24 66.2537

Fig. 2

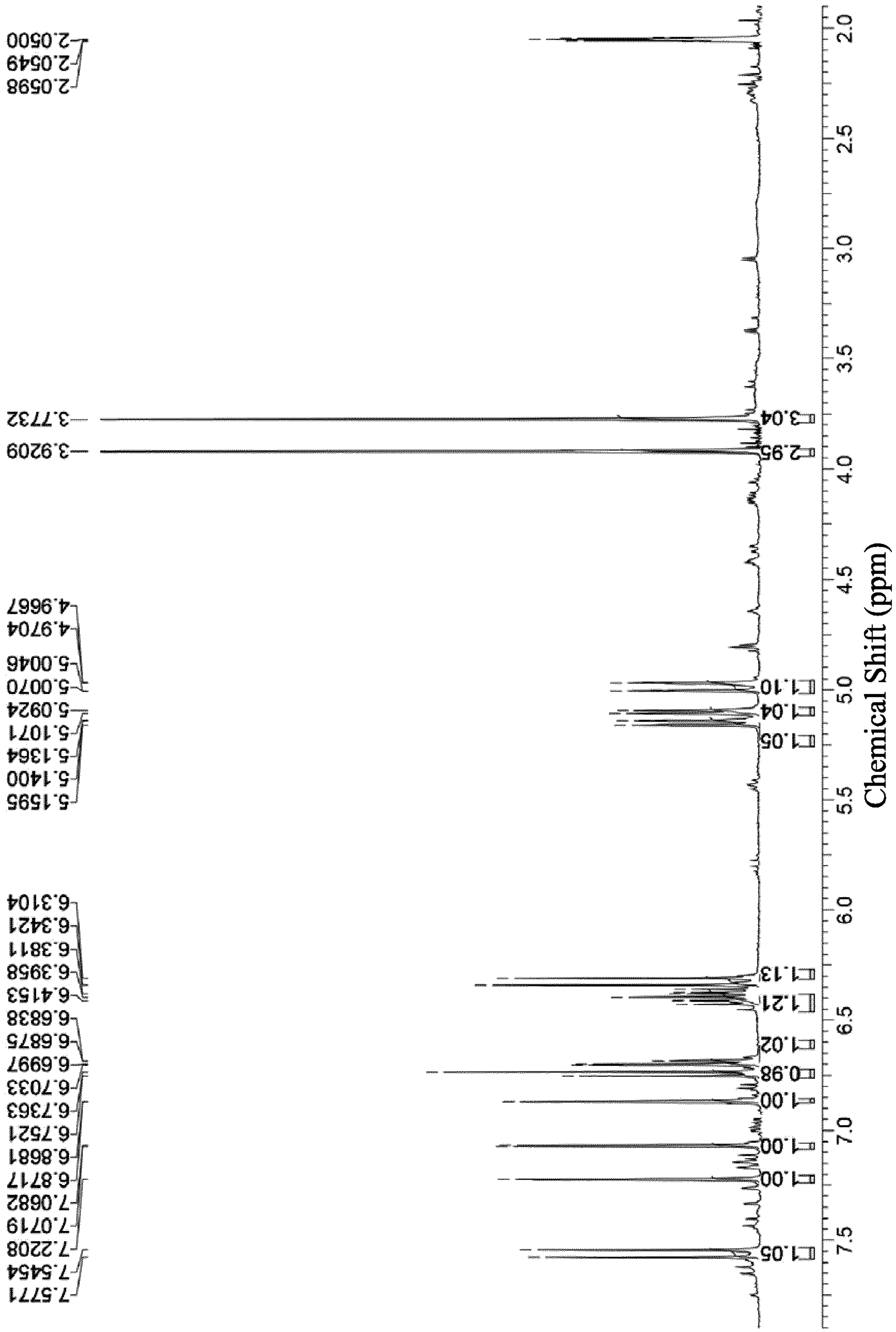


Fig. 3

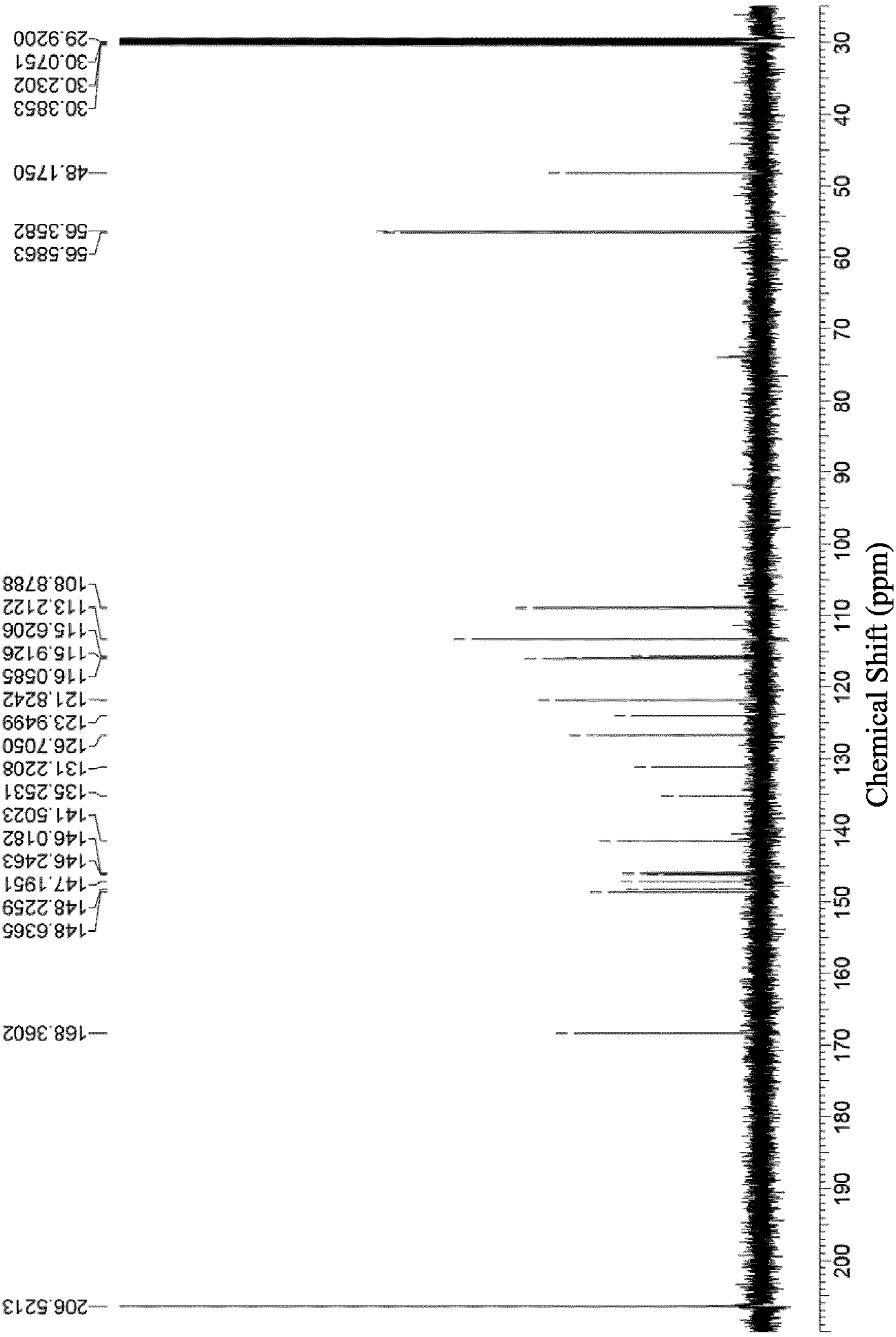
29.9200
30.0751
30.2302
30.3853
48.1750
56.3582
56.5863
108.8788
113.2122
115.6206
115.9126
116.0585
121.8242
123.9499
126.7050
131.2208
135.2531
141.5023
146.0182
146.2463
147.1951
148.2259
148.6365
168.3602
206.5213
30
40
50
60
70
80
90
100
110
120
130
140
150
160
170
180
190
200
Chemical Shift (ppm)

Fig. 4

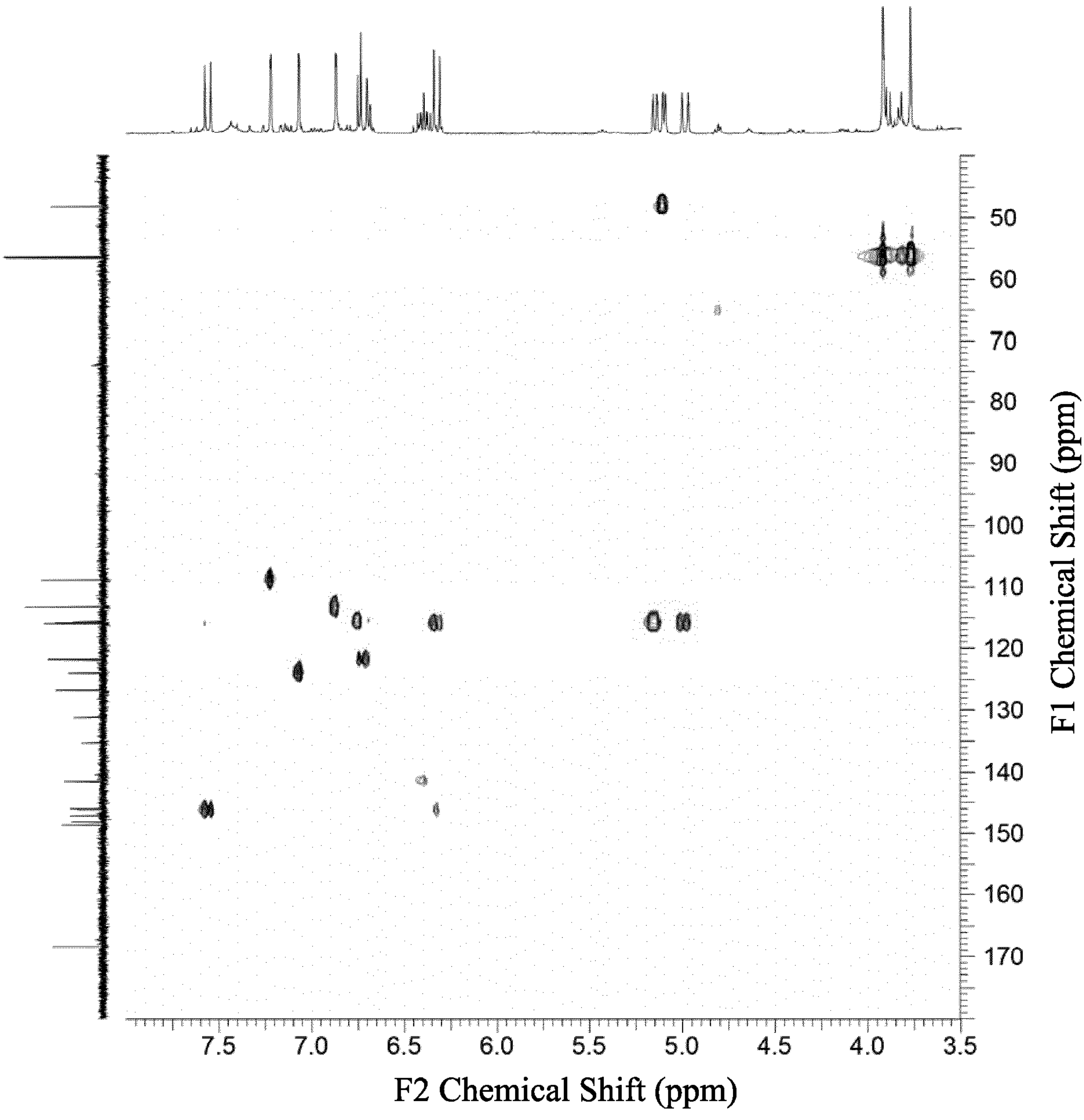
7.5
7.0
6.5
6.0
5.5
5.0
4.5
4.0
3.5
F2 Chemical Shift (ppm)
50
60
70
80
90
100
110
120
130
140
150
160
170
F1 Chemical Shift (ppm)

Fig. 5

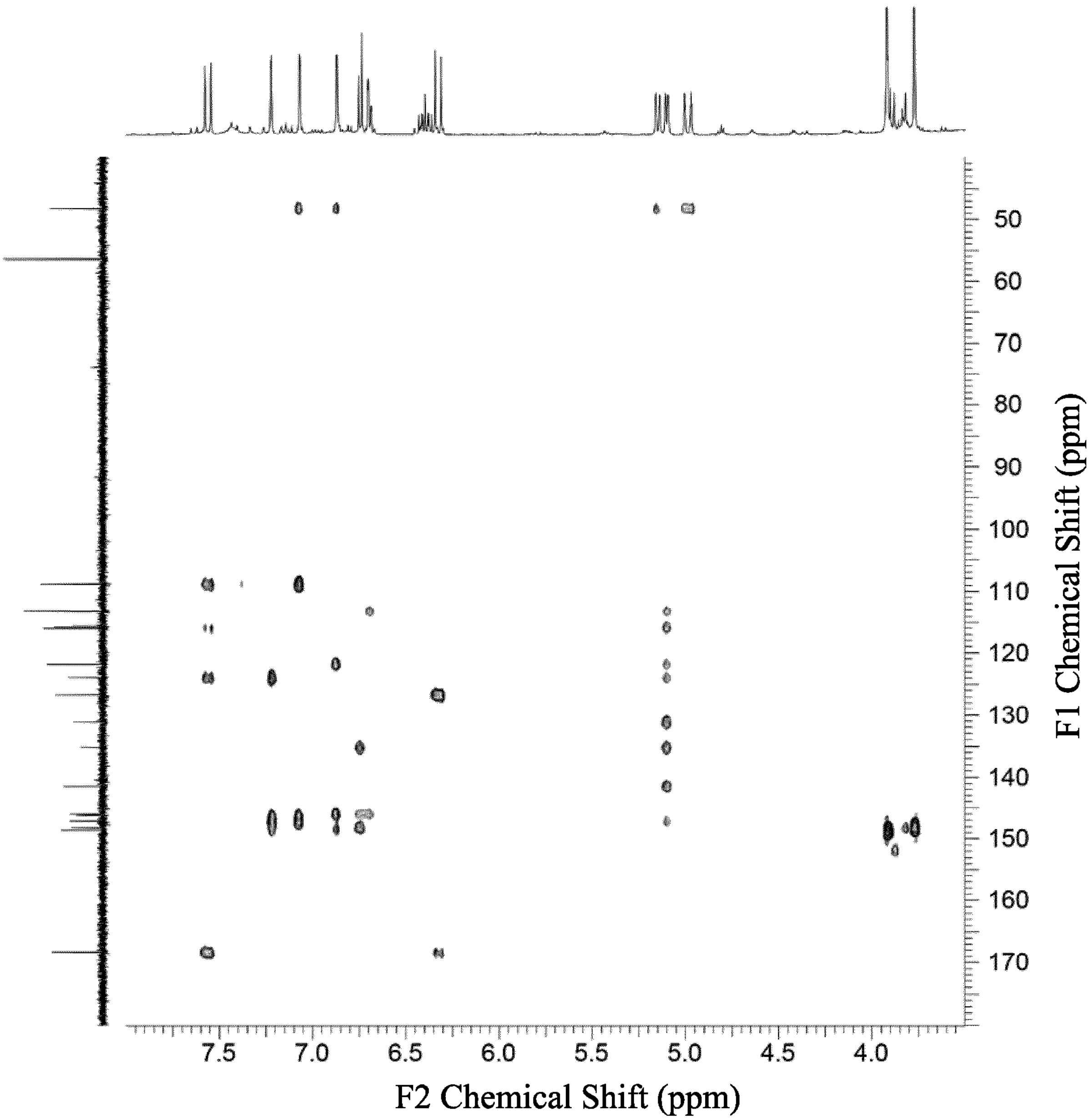
F1 Chemical Shift (ppm)
50
60
70
80
90
100
110
120
130
140
150
160
170
7.5
7.0
6.5
6.0
5.5
5.0
4.5
4.0
F2 Chemical Shift (ppm)

Fig. 6

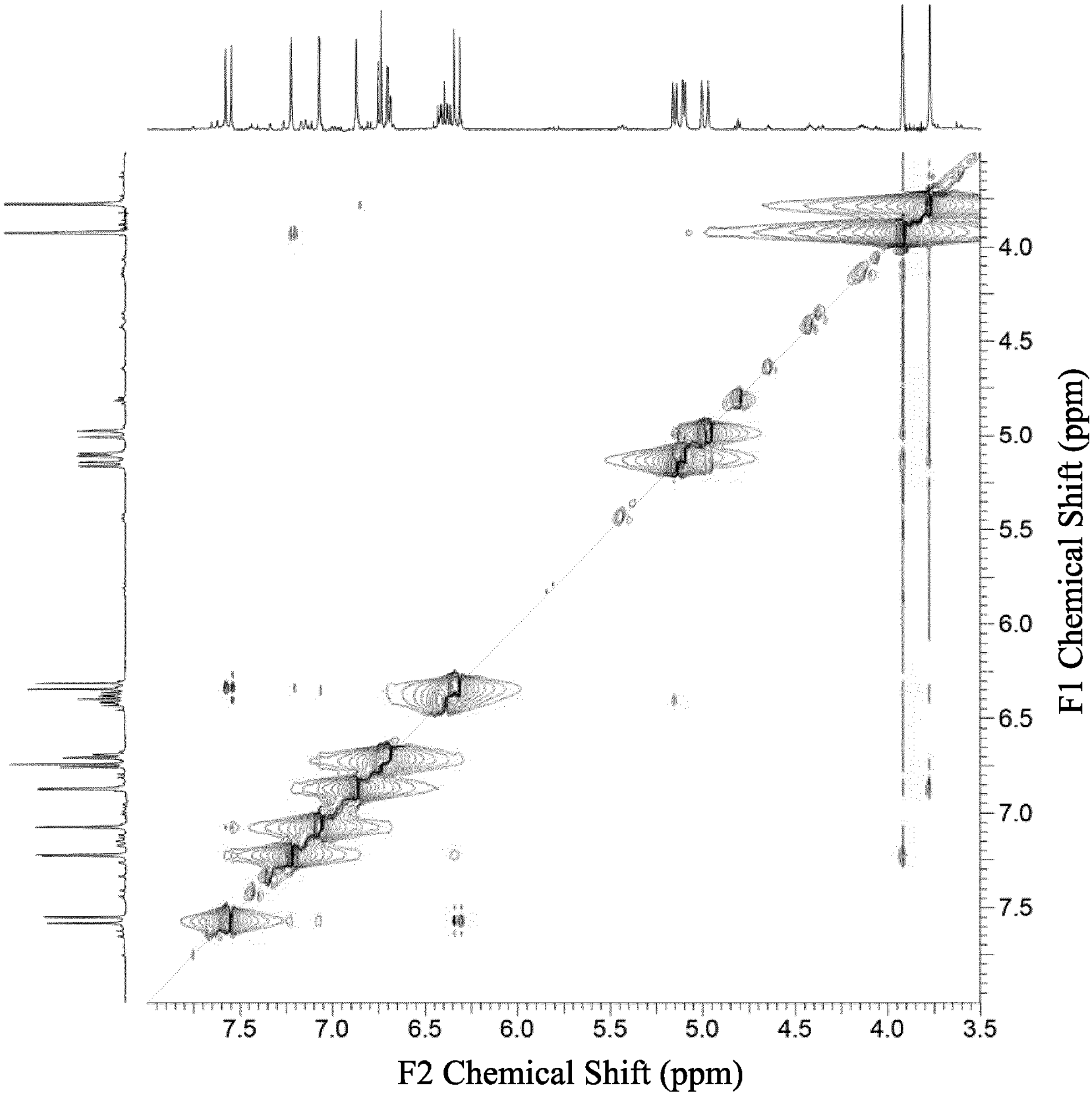
F1 Chemical Shift (ppm)
F2 Chemical Shift (ppm)
4.0
4.5
5.0
5.5
6.0
6.5
7.0
7.5
3.5

Fig. 7

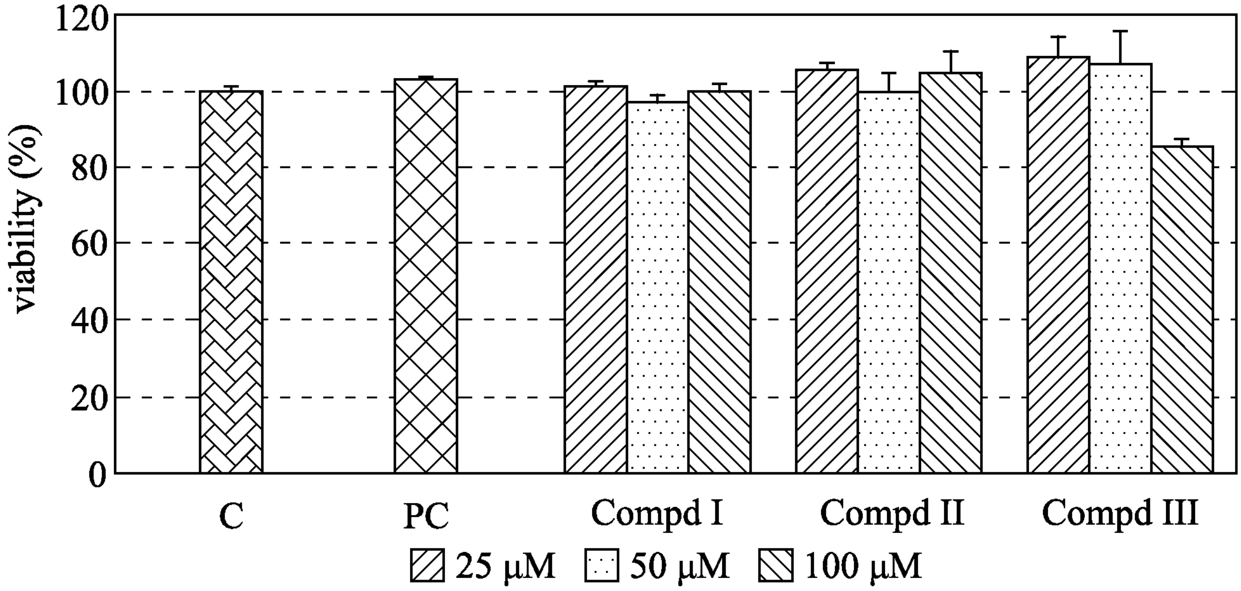
120
100
80
60
40
20
0
viability (%)
C
PC
Compd I
Compd II
Compd III
25 μM
50 μM
100 μM

Fig. 8A

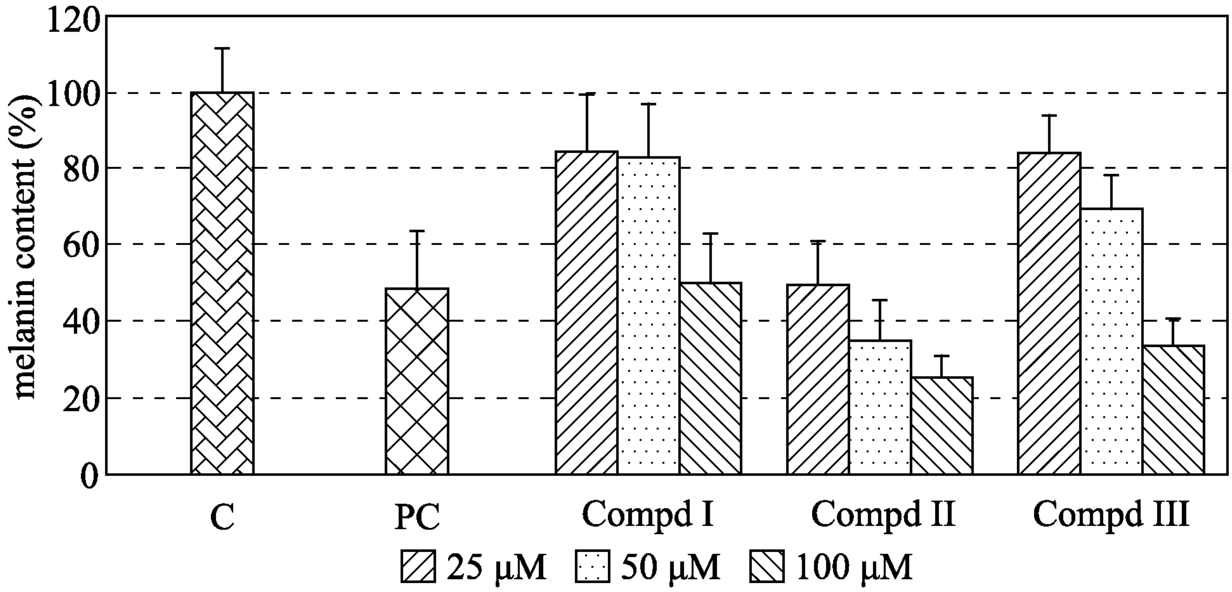
120
100
80
60
40
20
0
melanin content (%)
C
PC
Compd I
Compd II
Compd III
25 μM
50 μM
100 μM

Fig. 8B

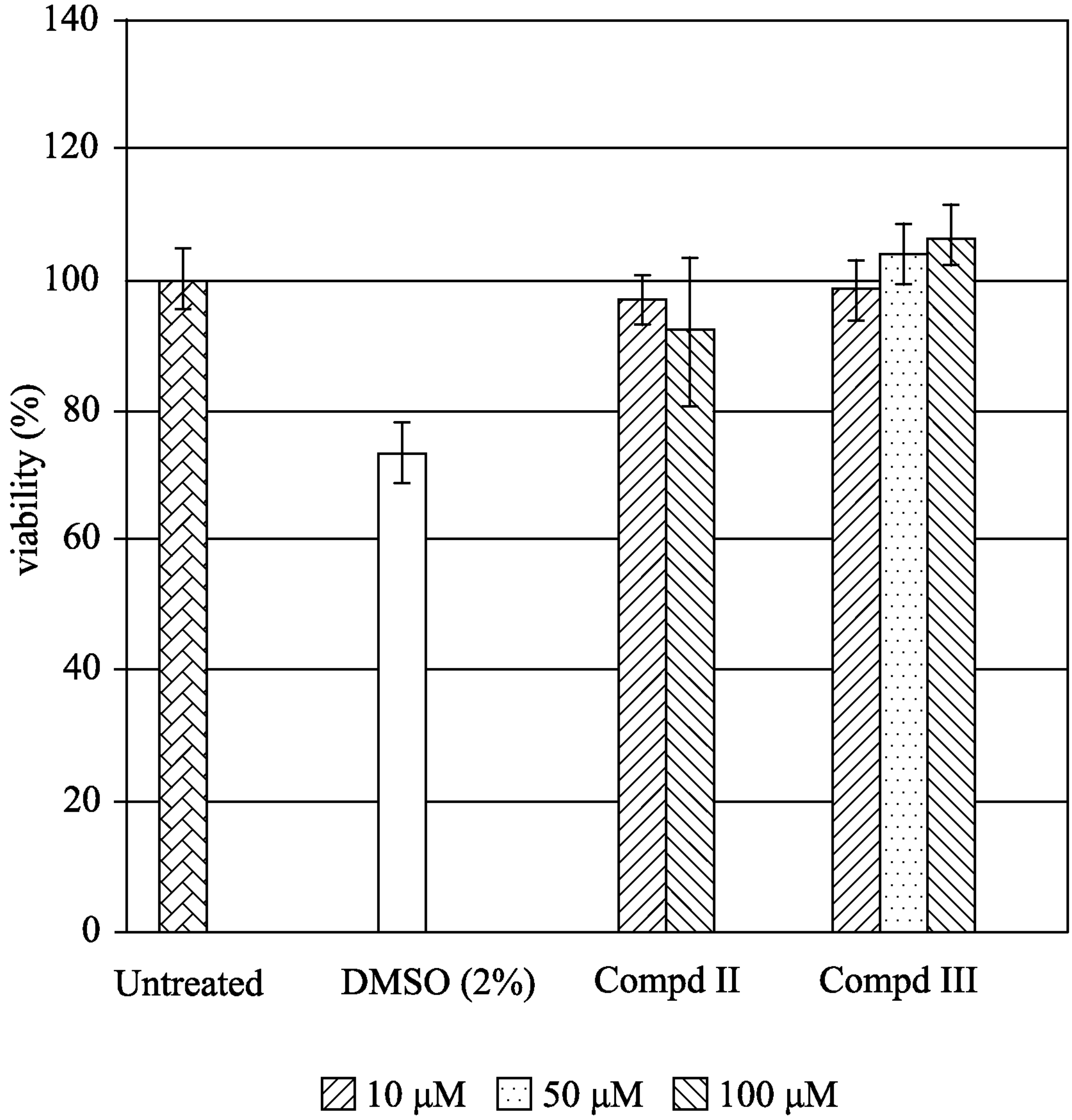
140
120
100
80
60
40
20
0
viability (%)
Untreated
DMSO (2%)
Compd II
Compd III
10 μM
50 μM
100 μM

Fig. 8C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2000247874 A

### Non-patent literature cited in the description


- **BARTSCHAT, D. ; BECK, T. ; MOSANDL, A.** Stereoisomeric flavor compounds. 79. Simultaneous enantioselective analysis of 3-butylphthalide and 3-butylhexahydrophthalide stereoisomers in celery, celeriac, and fennel. *Journal of Agricultural and Food Chemistry,* 1997, vol. 45, 4551-4557
- **OGURO, D. ; WATANABE, H.** Synthesis and sensory evaluation of all stereoisomers of sedanolide. *Tetrahedron,* 2011, vol. 67, 777-781
- **HOSOI J ; ABE E ; SUDA T ; KUROKI T.** Regulation of melanin synthesis of B16 mouse melanoma cells by 1 alpha, 25-dihydroxyvitamin D3 and retinoic acid. *Cancer Res.,* 1985, vol. 45 (4), 1474-8